# EUROPÄISCHE PATENTSCHRIFT

(11) **EP 1 095 024 B1**
(45) Veröffentlichungstag und Bekanntmachung des Hinweises auf die Patenterteilung: **19.11.2003**
(21) Anmeldenummer: 99939989.2
(22) Anmeldetag: 06.07.1999
(51) Int. Cl.: C07D 233/61

(54) **VERFAHREN ZUR HERSTELLUNG VON CARBONYLDIIMIDAZOLEN**
METHOD FOR PRODUCING CARBONYL DIIMIDAZOLES
PROCEDE DE PREPARATION DE DIIMIDAZOLES DE CARBONYLE

(30) Priorität: 08.07.1998 DE 19830556
(43) Veröffentlichungstag der Anmeldung: 02.05.2001
(73) Patentinhaber: BASF AKTIENGESELLSCHAFT, 67056 Ludwigshafen (DE)
(72) Erfinder: STAMM, Armin, D-55128 Mainz (DE); HENKELMANN, Jochem, D-68165 Mannheim (DE)
(74) Vertreter: Isenbruck, Günter, Dr.
(86) Internationale Anmeldenummer: EP9904729
(87) Internationale Veröffentlichungsnummer: WO00002863

(56) Entgegenhaltungen:
- EP-A- 0 692 476
- WO-A-98/31672
- US-A- 3 991 071
- US-A- 5 552 554
- WALTER W ET AL: "ZUR UMSETZUNG VON AZOLEN MIT ANORGANISCHEN SAEURECHLORIDEN" LIEBIGS ANNALEN DER CHEMIE, Nr. 11, 1. Januar 1979 (1979-01-01), Seiten 1756-1767, XP002064142 ISSN: 0170-2041

## Beschreibung

Die vorliegende Erfindung betrifft ein verbessertes Verfahren zur Herstellung von Carbonyldiimidazolen aus Imidazolen und Phosgen unter Vermeidung des Koppelproduktes Imidazol-Hydrochlorid.

Carbonyldiimidazol (CDI) ist ein häufig genutztes Reagenz zur Einführung von Carbonylgruppen, beispielsweise zur Herstellung von Carbonaten, Harnstoffen oder Urethanen beziehungsweise zur Aktivierung von unreaktiven Reaktanden in der Ester- oder Amidsynthese (Angew. Chem. 74 (1962), 407).

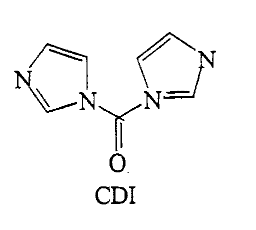

Grundsätzlich sind zwei Herstellwege für Carbonyldiimidazol bekannt. In Chem. Ber. 93 (1960), 2804 und US 4,965,366 wird ein zweistufiges Verfahren beschrieben, bei dem zunächst Imidazol mit Trimethylsilylchlorid zum 1-Trimethylsilylimidazol und dieses nachfolgend mit Phosgen nach folgendem Schema zum CDI umgesetzt wird.

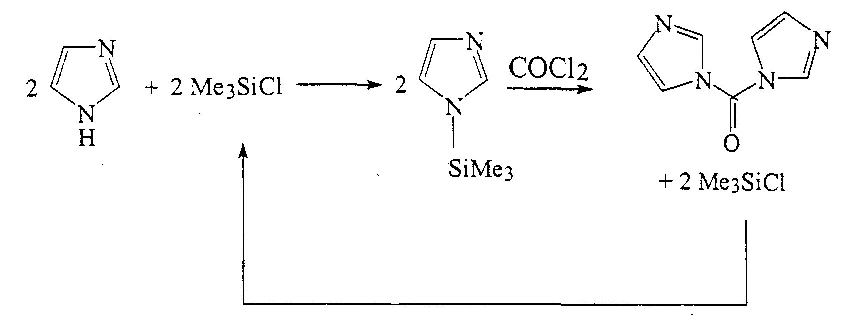

Obwohl das Trimethylsilylchlorid im Kreislauf wiederverwendet werden kann, erfordert diese Syntheseroute einen erheblichen Zeitaufwand und bewirkt damit eine schlechte Raum-Zeit-Ausbeute des Wertproduktes. Des weiteren stellt die Handhabung des hydrolyseempfindlichen Trimethylsilylchlorides zusätzliche Anforderungen an die Reaktionsapparaturen.

Der elegantere Weg zur Herstellung von CDI ist die ursprünglich von Staab et al. eingeführte und in diversen Veröffentlichungen beschriebene direkte Phosgenierung von Imidazol (Liebigs Ann. Chem. 609 (1957), 75; Chem. Ber. 96 (1963), 3374; Org. Synth. Coll. Vol V (1973), 201). Zwar entstehen dabei als Koppelprodukt zwei Mol Imidazol-Hydrochlorid pro Mol CDI, ersteres kann aber durch basische Aufarbeitung wieder in Imidazol umgewandelt und in die Phosgenierung zurückgeführt werden:

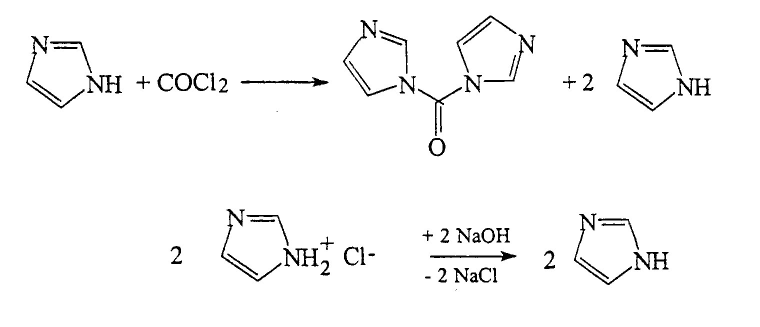

In EP-A-0 692 476 wird die in den vorangegangenenen Literaturstellen beschriebene Syntheseroute lediglich um eine Methode zur Entwässerung des Lösungsmittels erweitert. Eine erhebliche Schwierigkeit der Originalvorschriften bleibt jedoch bestehen. Das als Koppelprodukt mitgebildete Imidazol-Hydrochlorid muß bei hohen Temperaturen (80 bis 100°C) durch eine Filtration unter Feuchtigkeitsausschluß von der CDI enthaltenden Reaktionslösung abgetrennt werden. Zur Einhaltung einer genügend hohen Produktqualität muß ein hoher technischer Aufwand betrieben werden, da das CDI außerordentlich hydrolyseempfindlich ist.

Nachteilig an allen bisherigen Verfahren zur direkten Phosgenierung von Imidazol ist die Bildung des Koppelproduktes Imidazol-Hydrochlorid, das in einer zusätzlichen Verfahrensstufe zurückgeführt werden muß und wodurch die Raum-Zeit-Ausbeute halbiert wird.

Die prioritätsältere, nicht veröffentlichte WO 98/31672 betrifft ein Verfahren zur Herstellung von z.B. CDI durch Umsetzung von z.B. Imidazol mit Phosgen in Gegenwart einer organischen Base, z.B. Tri(n-butyl)amin.

Liebigs Ann. Chem. 1979, 1756 - 1767 betrifft die Umsetzung von Azolen mit organischen Säurechloriden. Auf Seite 1757 wird in der ersten Reaktionsgleichung die Umsetzung von Imidazol mit Phosgen beschrieben. Es wird angegeben, daß bei Verwendung von Thionylchlorid anstelle von Phosgen (Vorgehensweise 1b) eine äquimolare Menge Triethylamin zugegeben werden kann, wodurch der Imidazolanteil, der zur Bindung des Chlorwasserstoffs benötigt wird, entfällt. In den experimentellen Beispielen auf Seite 1761 wird jedoch nur eine Variante beschrieben, bei der Imidazol mit Schwefeldichlorid in Gegenwart von Triethylamin umgesetzt wird. Es wird in THF als Lösungsmittel gearbeitet.

Aufgabe der vorliegenden Erfindung ist die Bereitstellung eines Verfahrens zur Herstellung von Carbonyldiimidazolen aus Imidazolen und Phosgen, das die genannten Nachteile vermeidet.

Die Aufgabe wird erfindungsgemäß gelöst durch ein Verfahren zur Herstellung von Carbonyldiimidazolen der allgemeinen Formeln Ia, Ib, Ic oder Gemischen davon 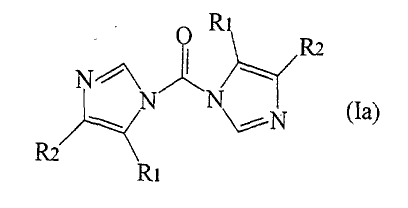 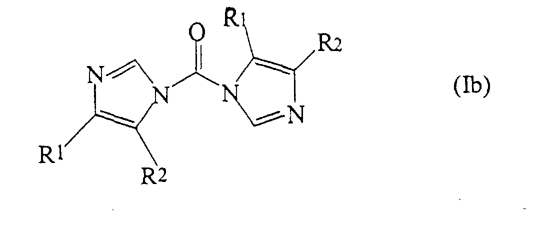 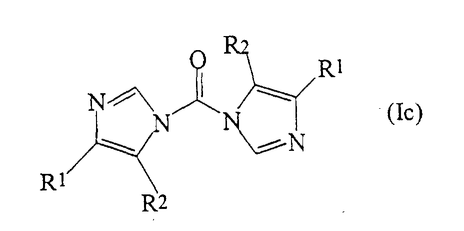 wobei R¹ Wasserstoff oder ein C₁₋₄-Alkylrest und R² Wasserstoff oder ein Methylrest ist durch Umsetzung mindestens eines Imidazols der allgemeinen Formeln IIa und IIb 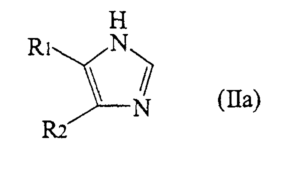 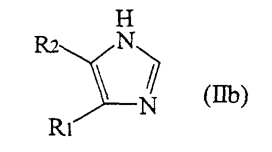 in denen R¹ und R² die vorstehend angegebenen Bedeutung haben, mit Phosgen in einem inerten Lösungsmittel, wobei man dem Reaktionsgemisch, bezogen auf die Imidazole, stöchiometrische Mengen oder einen Überschuß von bis zu 30 Mol-% einer organischen Stickstoffbase, die einen niedrigeren pK_{b}-Wert als Imidazol aufweist, zusetzt, wobei das gebildete Carbonyldiimidazol durch Abkühlen des Reaktionsgemisches auskristallisiert und als Feststoff abgetrennt wird, wobei das gebildete Hydrochlorid der Stickstoffbase in der organischen Lösung verbleibt, das Hydrochlorid der Stickstoffbase durch Neutralisation mit einer stärkeren wässrigen anorganischen Base in die freie Base zurückgeführt wird, die im organischen Lösungsmittel verbleibt, und die freigesetzte Stickstoffbase im organischen Lösungsmittel von der wässrigen Phase abgetrennt und wieder in die Synthese zurückgeführt wird, und daß als Stickstoffbase ein tertiäres Amin der allgemeinen Formel NR₃ eingesetzt wird, wobei R jeweils unabhängig voneinander verzweigte oder unverzweigte C₁₋₁₀-Alkylreste bedeuten oder zwei der Reste mit einem Stickstoffatom einen 5- oder 6-gliedrigen heterocycloaliphatischen oder heteroaromatischen Ring bilden.

Gemäß dem Verfahren wird die Bildung von Imidazol-Hydrochlorid durch die Zugabe einer organischen Base vermieden, so daß eine vollständige Umsetzung des eingesetzten Imidazols zum Carbonyldiimidazol erfolgen kann. Bei dieser Syntheseführung wird die als Beiprodukt zwangsläufig anfallende Salzsäure durch eine stärkere Base als Imidazol gebunden, so daß das gesamte eingesetzte Imidazol zum Carbonyldiimidazol abreagieren kann. Das Hydrochlorid der Hilfsbase muß dann nur noch vom Produkt getrennt und nach Überführen in die freie Base in die Synthese zurückgeführt werden.

Imidazol ist selbst eine schwache Base (pK_{b}-Wert etwa 7) und dient in der Phosgenierungsreaktion gleichzeitig als Reagenz und Säurefänger. Durch Zugabe einer stärkeren Stickstoffbase, beispielsweise eines tertiären aliphatischen Amins, insbesondere Tributylamin, bei der Synthese wird entweder gleich bevorzugt die stärkere Base protoniert oder aus dem gebildeten Hydrochlorid wieder Imidazol zurückgebildet:

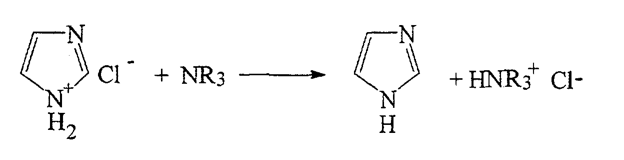

Das dadurch wieder als freie Base vorliegende Imidazol kann weiter phosgeniert werden. Um einen vollständigen Umsatz des gesamten eingesetzten Imidazols zu gewährleisten, ist gemäß der folgenden Bruttogleichung eine äquivalente Menge an tertiärer Stickstoffbase erforderlich.

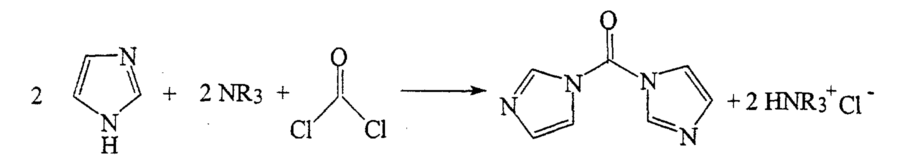

Der Vorteil des Verfahrens liegt unter anderem in einer doppelt so hohen Raum-Zeit-Ausbeute im Vergleich zu den bislang beschriebenen Verfahren, da das eingesetzte Imidazol komplett umgesetzt wird und nicht die Hälfte des Imidazols als Hydrochlorid ungenutzt zurückgeführt werden muß.

Die Umsetzung wird in einem inerten Lösungsmittel vorzugsweise bei einer Temperatur im Bereich von 60 bis 100°C durchgeführt, besonders bevorzugt bei 60 bis 80°C in einem substituierten aromatischen Kohlenwasserstoff Dabei werden bevorzugt Xylol oder Chlorbenzol eingesetzt, wobei Xylol als Gemisch von o-, mund p-Xylol oder als eines der Isomeren vorliegen kann. Besonders bevorzugt werden Xylol oder dessen Isomerengemische eingesetzt.

Als tertiäre Stickstoffbase mit einem niedrigeren pK_{b}-Wert als dem pK_{b}-Wert von Imidazol.

Wird ein tertiäres Amin der allgemeinen Formel NR₃ eingesetzt, wobei R jeweils unabhängig voneinander verzweigte oder unverzweigte C₁₋₁₀-Alkylreste bedeuten oder zwei der Reste mit einem Stickstoffatom einen 5- oder 6-gliedrigen heterocycloaliphatischen oder heteroaromatischen Ring bilden.

Vorzugsweise ist die Stickstoffbase ein tertiäres aliphatisches Amin, in dem die Reste R gleich sind und C₁₋₄-Alkylreste sind, wie Trimethyl-, Triethyl-, Tripropyl-, Triisopropyl-, Tri-n-butyl-, Triisobutyl-, Tri-sek-butylamin. Besonders bevorzugt ist Tri-n-butylamin. Beispiele für Amine, in denen zwei der Reste einen Ring aufspannen, sind N-Alkylpyrrolidine und N-Alkylpiperidine.

Im folgenden wird die Vorgehensweise am Beispiel von Carbonyldiimidazol (CDI) beschrieben. Das Verfahren ist jedoch auch für die vorstehend genannten substituierten Verbindungen einsetzbar.

Erwartungsgemäß sollte das gebildete Amin-Hydrochlorid aus dem Reaktionsgemisch als feste Phase ausfallen. Im beschriebenen Verfahren bleibt die Reaktionslösung bei höheren Temperaturen (50 bis 60°C) homogen. Erst beim Abkühlen fällt Carbonyldi-imidazol in farblosen Nadeln aus der Reaktionsmischung aus, während das Tributylamin-Hydrochlorid vollständig im Lösungsmittel verbleibt. Ohne an eine Theorie gebunden zu sein, wird offenbar das CDI durch das Amin-Hydrochlorid durch einen Aussalzeffekt aus der Lösung gedrängt, während das Tributylamin-Hydrochlorid aufgrund seiner längeren und apolaren Alkylreste im Lösungsmittel gelöst bleibt.

Das gebildete CDI wird durch Abkühlen des Reaktionsgemisches auskristallisiert und als Feststoff abgetrennt, wobei das gebildete Hydrochlorid der Stickstoffbase in der organischen Lösung verbleibt. Das ausgefallene CDI kann durch einfaches Absaugen unter Inertgas oder durch Abtrennung mit Hilfe einer Zentrifuge vom Lösungsmittel/Amin-Hydrochlorid-Gemisch abgetrennt werden. Es ist bereits ohne Wäsche ausreichend rein für weitere Anwendungen. Das bedeutet einen erheblichen Verfahrensvorteil, da das Produkt, das sehr feuchtigkeitsempfindlich ist, nur einmal mit Luft in Berührung kommt. Das im Lösungsmittel gelöste Hydrochlorid des tertiären Amins wird durch Neutralisation mit einer stärkeren wässrigen anorganischen Base, vorzugsweise einer wässrigen Lösung von NaOH oder KOH, in die freie Base zurückgeführt, die im organischen Lösungsmittel verbleibt. Die freigesetzte Stickstoffbase im organischen Lösungsmittel wird sodann von der wässrigen Phase abgetrennt und wieder in die Synthese zurückgeführt.

Die Erfindung wird nachstehend anhand von Beispielen näher erläutert.

### Beispiel 1: (Referenz)

Alle Reaktionen werden in einem 2 l fassenden HWS-Gefäß mit Doppelmantelbeheizung sowie einem Kohlensäurekühler (-78°C) und Rührer durchgeführt.

185,4 g Tributylamin (1 mol) werden in 1010 g Xylol gelöst. Die Lösung wird zum Rückfluß erhitzt, und es wird solange Xylol an einem Wasserabscheider abdestilliert, bis kein Wasser mehr nachläuft (insgesamt ca. 10 ml). Nach Zugabe von 68 g (1 mol) Imidazol zur Reaktionsmischung werden innerhalb von 30 Minuten bei 68 bis 80°C insgesamt 51 g (0,5 mol) Phosgen eingegast. Nach dem Ende der Reaktion läßt man 60 Minuten bei 65°C nachreagieren. Der homogene Reaktionsaustrag wird in einen mit trockenem Argon gespülten Erlenmeyerkolben abgelassen. Nach Abkühlen bei Raumtemperatur fallen nach kurzer Zeit farblose Kristalle aus, die unter Argon abgesaugt und zweimal mit je 50 ml getrocknetem Xylol gewaschen werden. Nach Trocknung im Vakuum erhält man 59 g CDI vom Schmelzpunkt 112°C.

| | | | | |
|---|---|---|---|---|
| Elementaranalyse | berechnet | C: 51,84 | H: 3,74 | N: 34,56 |
| | gefunden | C: 51,90 | H: 3,80 | N: 34,60 |

### Beispiel 2:

### Rückführung des Tributylamin-Hydrochlorides mit erneuter Umsetzung

Zur Mutterlauge aus Beispiel 1 wird solange 40%-ige Natronlauge unter Rühren zugegeben, bis der pH-Wert bei 12 konstant bleibt (insgesamt: g). Die wässrige (untere) Phase wird abgetrennt, die xylolische Lösung von Tributylamin (Oberphase) wird in das Reaktionsgefäß überführt, und es wird solange am Wasserabscheider Xylol abdestilliert, bis sich kein Wasser mehr abscheidet. Zur Reaktionsmischung gibt man nach Abkühlen 68 g (1 mol) Xylol und 21 g (0,1 mol) Tributylamin.

Bei 72 bis 83°C werden innerhalb von 30 Minuten insgesamt 51 g (0,5 mol) Phosgen eingegast. Nach Ende der Zudosierung von Phosgen wird 60 Minuten bei 65°C nachgerührt. Die Aufarbeitung wird wie in Beispiel 1 durchgeführt. Nach Trocknen erhält man 57 g CDI vom Schmelzpunkt 112°C.

### Beispiel 3:

Die Mutterlauge aus Beispiel 2 wird analog dem in Beispiel 2 beschriebenen Vorgehen aufgearbeitet und bei 70 bis 79°C phosgeniert.
Nach Aufarbeitung erhält man 53 g CDI vom Schmelzpunkt 109°C.

### Beispiel 4: (Wiederholung von Beispiel 1)

185,4 g Tributylamin (1 mol) werden in 1010 g Xylol gelöst. Die Lösung wird zum Rückfluß erhitzt, und es wird solange Xylol an einem Wasserabscheider abdestilliert, bis kein Wasser mehr nachläuft (insgesamt ca. 10 ml). Nach Zugabe von 68 g (1 mol) Imidazol zur Reaktionsmischung werden innerhalb von 30 Minuten bei 68 bis 78°C insgesamt 56 g (0,57 mol) Phosgen eingegast. Nach dem Ende der Reaktion läßt man 60 Minuten bei 65°C nachreagieren. Der homogene Reaktionsaustrag wird in einen mit trockenem Argon gespülten Erlenmeyerkolben abgelassen. Nach Abkühlen bei Raumtemperatur fallen nach kurzer Zeit farblose Kristalle aus, die unter Argon abgesaugt und zweimal mit je 50 ml getrocknetem Xylol gewaschen werden. Nach Trocknung im Vakuum erhält man 62 g CDI vom Schmelzpunkt 114°C.

### Beispiel 5:

Die Synthese wid entsprechend Beispielen 1 und 4 mit Chlorbenzol als Lösungsmittel durchgeführt. Bei identischer Fahrweise und Aufarbeitung erhält man 50 g CDI vom Schmelzpunkt 114°C.

## Patentansprüche

1. Verfahren zur Herstellung von Carbonyldiimidazolen der allgemeinen Formeln Ia, Ib, Ic oder Gemischen davon 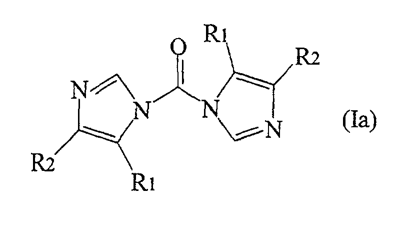 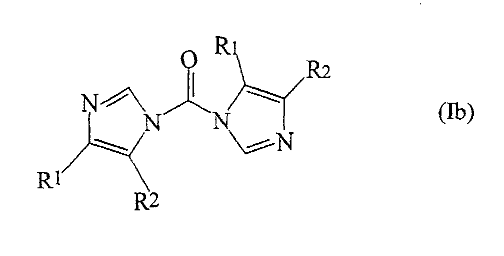 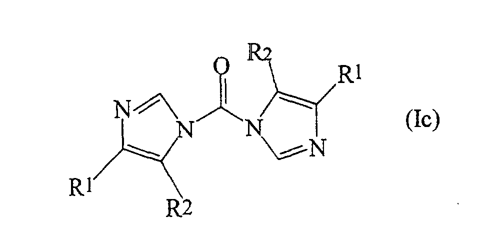 wobei R¹ Wasserstoff oder ein C₁₋₄-Alkylrest und R² Wasserstoff oder ein Methylrest, ist durch Umsetzung mindestens eines Imidazols der allgemeinen Formeln IIa und IIb 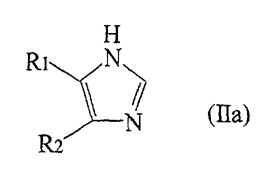 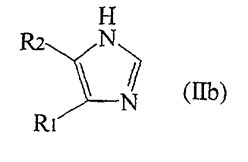 in denen R¹ und R² die vorstehend angegebene Bedeutung haben,
mit Phosgen in einem inerten Lösungsmittel, wobei man dem Reaktionsgemisch, bezogen auf die Imidazole, stöchiometrische Mengen oder einen Überschuß von bis zu 30 Mo.-% einer organischen Stickstoffbase, die einen niedrigeren pK_{b}-Wert als Imidazol aufweist, zusetzt, **dadurch gekennzeichnet, daß** das gebildete Carbonyldiimidazol durch Abkühlen des Reaktionsgemisches auskristallisiert und als Feststoff abgetrennt wird, wobei das gebildete Hydrochlorid der Stickstoffbase in der organischen Lösung verbleibt, das Hydrochlorid der Stickstoffbase durch Neutralisation mit einer stärkeren wässrigen anorganischen Base in die freie Base zurückgeführt wird, die im organischen Lösungsmittel verbleibt, und die freigesetzte Stickstoffbase im organischen Lösungsmittel von der wässrigen Phase abgetrennt und wieder in die Synthese zurückgeführt wird und daß als Stickstoffbase ein tertiäres Amin der allgemeinen Formel NR₃ eingesetzt wird, wobei R jeweils unabhängig voneinander verzweigte oder unverzweigte C₁₋₁₀-Alkylreste bedeuten oder zwei der Reste mit einem Stickstoffatom einen 5- oder 6-gliedrigen heterocycloaliphatischen oder heteroaromatischen Ring bilden.

2. Verfahren nach Anspruch 1, **dadurch gekennzeichnet, daß** die Stickstoffbase ein tertiäres aliphatisches Amin ist, in dem die Reste R gleich sind und C₁₋₄-Alkylreste sind.

3. Verfahren nach Anspruch 2, **dadurch gekennzeichnet, daß** das tertiäre aliphatische Amin Tri-n-butylamin ist.

4. Verfahren nach einem der Ansprüche 1 bis 3, **dadurch gekennzeichnet, daß** als Lösungsmittel ein substituierter aromatischer Kohlenwasserstoff verwendet wird.

5. Verfahren nach Anspruch 4, **dadurch gekennzeichnet, daß** als Lösungsmittel o-Xylol oder Isomerenmische von o, m und p-Xylol verwendet werden.

## Claims

1. A process for preparing carbonyldiimidazoles of the general formulae Ia, Ib, Ic or mixtures thereof 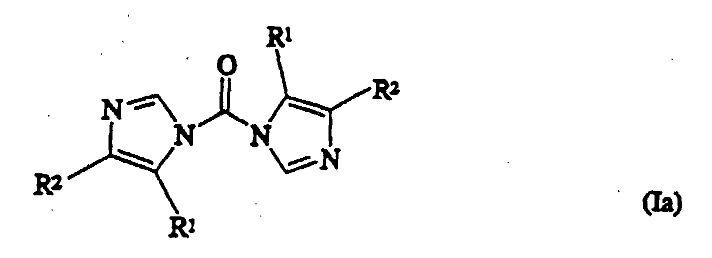 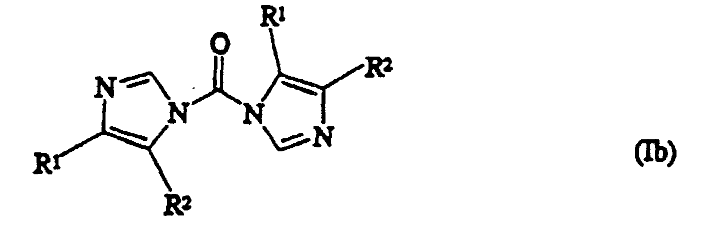 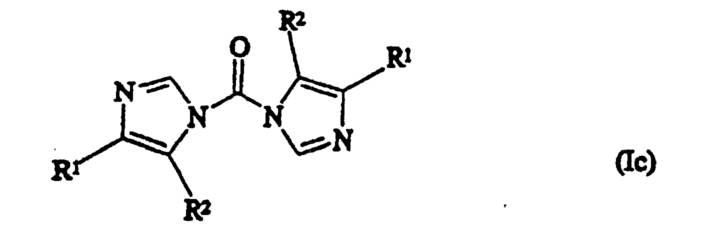 where R¹ is hydrogen or a C₁₋₄-alkyl radical and R² is hydrogen or a methyl radical, by reacting at least one imidazole of the general formulae IIa and IIb 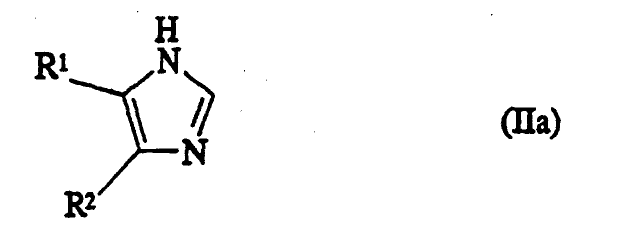 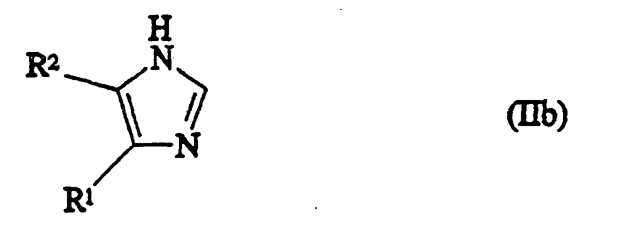 in which R¹ and R² have the abovementioned meaning, where stoichiometric amounts or an excess of up to 30 mol-% of an organic nitrogen base which has a lower pK_{b} than imidazole are added to the reaction mixture, based on the imidazoles, wherein the carbonyldiimidazole which is formed is crystallized out by cooling the reaction mixture and is removed as solid, while the hydrochloride which is formed from the nitrogen base remains in the organic solution, the hydrochloride of the nitrogen base is neutralized with a stronger aqueous organic base to return to the free base, which remains in the organic solvent, and the liberated nitrogen base in the organic solvent is separated from the aqueous phase and recycled to the synthesis, and wherein the nitrogen base employed is a tertiary amine of the general formula NR₃ where the R radicals are each, independently of one another, branched or unbranched C₁₋₁₀-alkyl, or two of the radicals form with one nitrogen atom a 5- or 6-membered heterocycloaliphatic or heteroaromatic ring.

2. A process as claimed in claim 1, wherein the nitrogen base is a tertiary aliphatic amine in which the R radicals are identical and are C₁₋₄-alkyl radicals .

3. A process as claimed in claim 2, wherein the tertiary aliphatic amine is tri-n-butylamine.

4. A process as claimed in any of claims 1 to 3, wherein a substituted aromatic hydrocarbon is used as solvent.

5. A process as claimed in claim 4, wherein o-xylene or mixtures of o, m and p-xylene isomers are used as solvent.

## Revendications

1. Procédé de préparation de carbonyldiimidazoles ayant les formules générales Ia, Ib, Ic, ou de mélanges de ces derniers 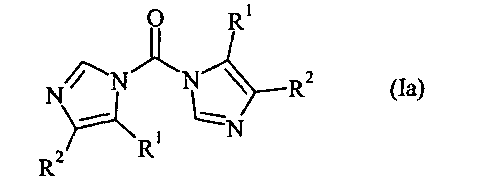 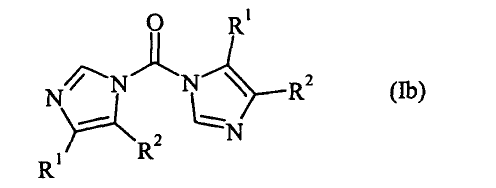 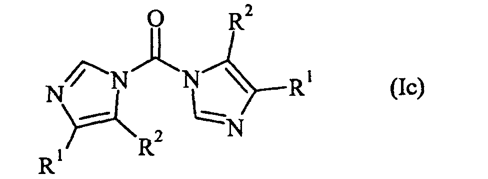 dans lesquelles R¹ est un atome d'hydrogène ou un groupe alkyle en C₁₋₄, et R² est un atome d'hydrogène ou un groupe méthyle, par réaction d'au moins un imidazole de formules générales IIa et IIb 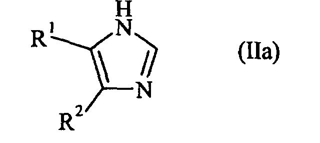 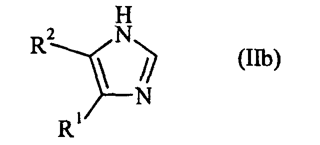 dans lesquelles R¹ et R² ont les significations données ci-dessus,
avec du phosgène dans un solvant inerte, ce pour quoi on ajoute au mélange réactionnel, par rapport aux imidazoles, des quantités stoechiométriques ou un excès allant jusqu'à 30 % en moles d'une base azotée organique, qui a un pK_{b} inférieur à celui de l'imidazole, **caractérisé en ce que** le carbonyldiimidazole formé est séparé par cristallisation par refroidissement du mélange réactionnel et est séparé sous forme d'un solide, le chlorhydrate de la base azotée ainsi formé restant dans la solution organique, le chlorhydrate de la base azotée étant reconverti en la base libre par neutralisation avec une base inorganique aqueuse plus forte, cette base restant dans le solvant organique, et la base azotée libérée étant dans le solvant organique séparée de la phase aqueuse et renvoyée dans la synthèse, et **en ce qu'**on utilise en tant que base azotée une amine tertiaire de formule générale NR₃ dans laquelle chacun des radicaux R représente indépendamment des autres un groupe alkyle en C₁₋₁₀ à chaîne droite ou ramifiée, ou encore deux des radicaux forment avec un atome d'azote un noyau hétérocycloaliphatique ou hétéroaromatique à 5 ou 6 chaînons.

2. Procédé selon la revendication 1, **caractérisé en ce que** la base azotée est une amine aliphatique tertiaire dans laquelle les radicaux R sont identiques et représentent des groupes alkyle en C₁₋₄.

3. Procédé selon la revendication 2, **caractérisé en ce que** l'amine aliphatique tertiaire est la tri-n-butylamine.

4. Procédé selon l'une des revendications 1 à 3, **caractérisé en ce qu'**on utilise en tant que solvant un hydrocarbure aromatique substitué.

5. Procédé selon la revendication 4, **caractérisé en ce qu'**on utilise en tant que solvant de l'o-xylène ou des mélanges d'isomères d'o-, de m- et de p-xylène.
